# EUROPEAN PATENT SPECIFICATION

(11) **EP 4 249 906 B1**
(45) Date of publication and mention of the grant of the patent: **31.12.2025**
(21) Application number: 23162854.6
(22) Date of filing: 20.03.2023
(51) Int. Cl.: G01N 30/86, G01N 30/88, G01N 33/49, G01N 33/72

(54) **METHOD OF MEASURING HEMOGLOBIN F**
VERFAHREN ZUR MESSUNG VON HÄMOGLOBIN F
PROCÉDÉ DE MESURE DE L'HÉMOGLOBINE F

(30) Priority: 22.03.2022 JP 2022045898
(43) Date of publication of application: 27.09.2023
(73) Proprietor: ARKRAY, Inc., Kyoto-shi, Kyoto 601-8045 (JP)
(72) Inventor: ISHIKAWA, Kazuki, Kyoto, 602-0008 (JP)
(74) Representative: Dehns

(56) References cited:
- JP-A- 2014 235 023
- JP-A- 2019 060 653
- KOSKINEN L ET AL: "Determination of fetal hemoglobin by cation exchange liquid chromatography", CLINICA CHIMICA ACTA, ELSEVIER BV, AMSTERDAM, NL, vol. 206, no. 3, 31 March 1992 (1992-03-31), pages 215 - 223, XP024785180, ISSN: 0009-8981, [retrieved on 19920331], DOI: 10.1016/0009-8981(92)90091-4

## Description

### BACKGROUND

### Technical Field

The present invention relates to a method of measuring hemoglobin F in a blood sample.

### Related Art

Hemoglobin in blood samples can be measured by separation/fractionation methods such as high-performance liquid chromatography or capillary electrophoresis. Since a measured value of hemoglobin F (HbF), which is a type of hemoglobin, can be used as a basis for diagnosis of hemoglobinopathy and thalassemia, the value needs to be highly accurate. Measurement of HbF is often carried out by capillary electrophoresis, and a measured value of HbF obtained by measurement by capillary electrophoresis is often used as a basis for diagnosis. Examples of a method of measuring hemoglobin F using liquid chromatography include the method disclosed in Japanese Patent Application Laid-Open (JP-A) No. 2014-235023.

JP 2019-060653 A discloses correcting the measurement of a ratio of the hemoglobin F peak to all hemoglobin peaks using a correction coefficient determined by measuring the ratio of the hemoglobin F peak to all hemoglobin peaks of samples with a known ratio.

"Determination of fetal haemoglobin by cation exchange liquid chromatography" by Koskinen et al. (Clinica Chimica Acta, vol. 206, no. 3, pages 215 to 223, 31 March 1992) discloses measuring hemoglobin F using cation exchange chromatography.

The component peaks found in a chromatogram of hemoglobin obtained by liquid chromatography show almost the same ratios as those of the component peaks found in an analysis chart of hemoglobin obtained by capillary electrophoresis, and only the HbF peak presents a difference. More specifically, the ratio of the HbF peak in liquid chromatography is lower than the ratio of the HbF peak in capillary electrophoresis.

In view of this, an aspect of the present disclosure provides a technique that enables a more accurate measurement of HbF from a chromatogram obtained by subjecting a blood sample to liquid chromatography.

### SUMMARY

The invention provides a method of measuring hemoglobin F, the method comprising: measuring, for a series of samples, a hemoglobin F peak value obtained by liquid chromatography and a hemoglobin F peak value obtained by capillary electrophoresis; calculating a predetermined factor corresponding to the hemoglobin F peak value obtained by capillary electrophoresis divided by the hemoglobin F peak value obtained by liquid chromatography; calculating, from a chromatogram obtained by subjecting a blood sample to liquid chromatography, a ratio of a peak value of a hemoglobin F peak (20) to a peak value of an entire hemoglobin peak; and calculating a corrected value of the hemoglobin F peak (20) with respect to the entire hemoglobin peak by multiplying the ratio by the predetermined factor.

According to an aspect of the invention, HbF can be more accurately measured from a chromatograph obtained by subjecting a blood sample to liquid chromatography.

### BRIEF DESCRIPTION OF THE DRAWINGS

Exemplary embodiments will be described in detail based on the following figures, wherein:
Fig. 1 schematically shows an example of a chromatograph of hemoglobin obtained by subjecting a blood sample to high-performance liquid chromatography;
Fig. 2 schematically shows a relationship between an HbF peak and a composite peak in a normal sample;
Fig. 3 schematically shows a relationship between an HbF peak and a composite peak in a high-HbF sample;
Fig. 4 schematically shows an analysis chart of hemoglobin obtained by subjecting a normal sample to capillary electrophoresis;
Fig. 5 schematically shows an analysis chart of hemoglobin obtained by subjecting a high-HbF sample to capillary electrophoresis;
Fig. 6 is an example of a chromatogram obtained using an Apparatus 1 by a first mode;
Fig. 7 is an example of a chromatogram obtained using an Apparatus 1 by a second mode;
Fig. 8 is an example of a chromatogram of a blood sample, obtained using an Apparatus 2;
Fig. 9 is an example of a chromatogram of a blood sample, obtained using an Apparatus 3 by a first mode;
Fig. 10 is an example of a chromatogram of a blood sample, obtained using an Apparatus 3 by a second mode;
Fig. 11 is an example of an analysis chart of a blood sample, obtained using a capillary electrophoresis apparatus; and
Fig. 12 is a graph illustrating a correlation between an HbF peak value obtained by liquid chromatography, and an HbF peak value obtained by capillary electrophoresis.

### DETAILED DESCRIPTION

Embodiments in the disclosure are described below with reference to drawings. The symbols shared among the diagrams represent identical portions even without any description. The "peak value" in the disclosure means the height or area of each peak found in a chromatogram, and the value may be either a relative value or an absolute value. The relative value may be the ratio to the total area of the chromatogram, may be the ratio to the total area of the peaks related to hemoglobin in the chromatogram, or may be the ratio to the area of a specific peak (such as an HbA0 peak).

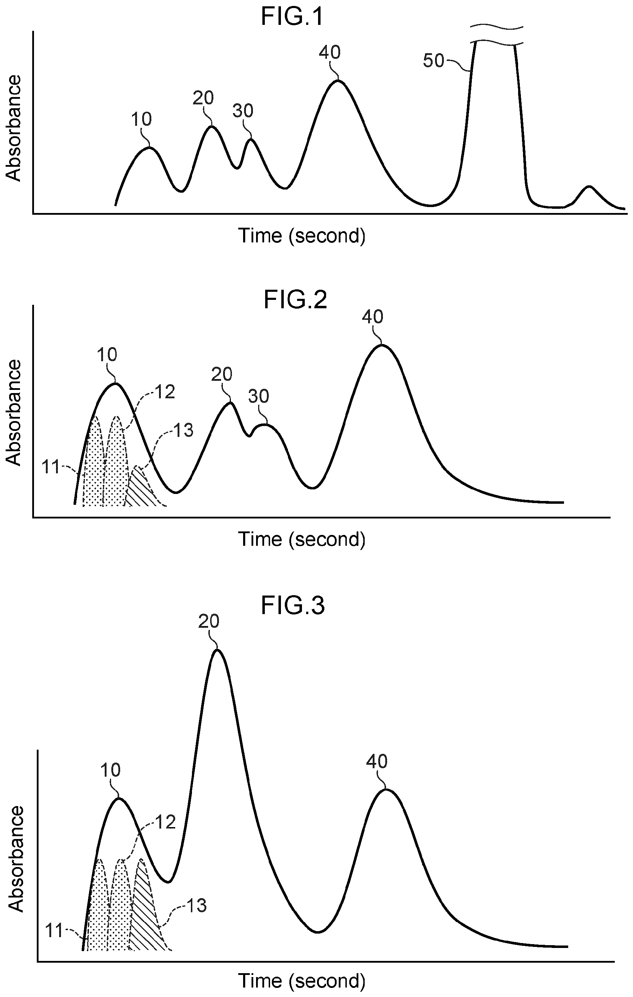

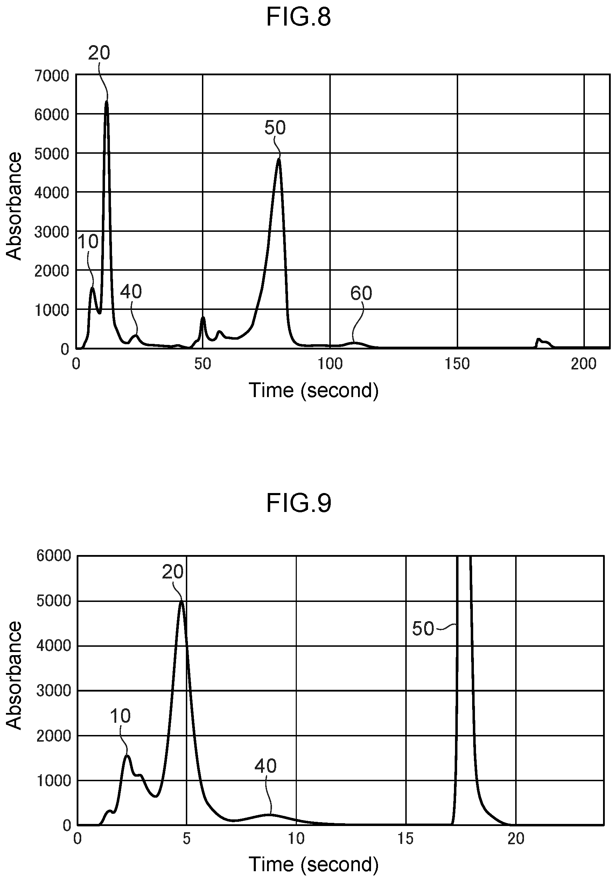

When a blood sample is subjected to liquid chromatography, a chromatograph of hemoglobin such as the one schematically represented in Fig. 1 can be obtained. In this chromatograph, a composite peak 10 including an HbA1a peak 11 and an HbA1b peak 12 (see Fig. 2 and Fig. 3), an HbF peak 20, an unstable HbA1c peak 30, an HbA1c peak 40, an HbA0 peak 50, and an HbA2 peak 60 (see Fig. 8) appear in the order of increasing rate of elution from the column.

Fig. 2 schematically shows a chromatograph of a normal blood sample having an HbF peak value of less than 1%, and Fig. 3 schematically shows a chromatograph of a blood sample of a high-HbF patient. As can be seen by comparison between these figures, which show HbA1c peaks 30 of almost the same size, the HbF peak 20 in the chromatograph of Fig. 3 is higher than the HbF peak 20 in the chromatograph of Fig. 2. In this case, the composite peak 10 in the chromatograph of Fig. 3 is also higher than the composite peak 10 in the chromatograph of Fig. 2.

Here, as shown in Fig. 2, the composite peak 10 includes an HbA1a peak 11 and an HbA1b peak 12 of HbA1, which is a glycosylated product of HbA. As shown in Fig. 3, an increase in the HbF peak 20 results in an increase in the composite peak 10. Since the composite peak 10 includes the HbA1a peak 11 and the HbA1b peak 12, which are glycosylated products, the increase in the composite peak 10 is thought to be due to an increase in a modified-HbF peak 13, which is a modified product of HbF, as shown in Fig. 3. In contrast, the normal sample shown in Fig. 2 hardly includes the modified-HbF peak 13, and most part of the composite peak 10 is thought to be formed by the HbA1a peak 11 and the HbA1b peak 12.

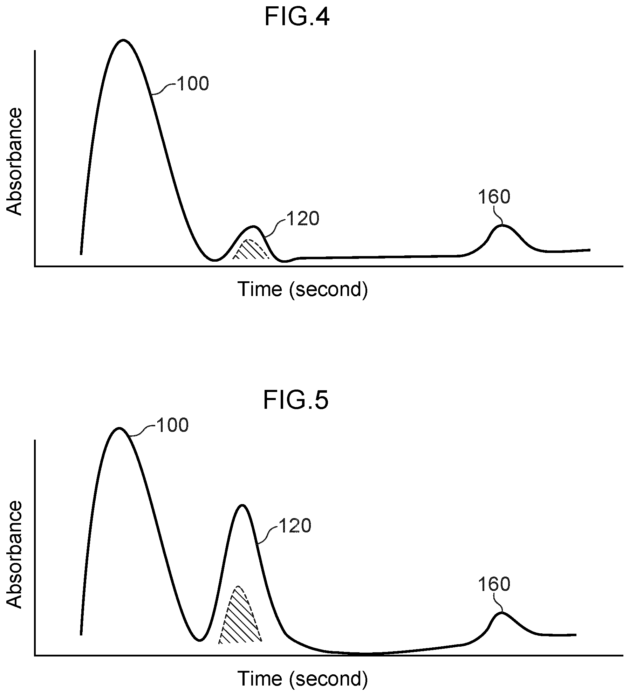

Fig. 4 schematically shows an analysis chart obtained by subjecting a normal blood sample having an HbF peak value of less than 1% to capillary electrophoresis, and Fig. 5 schematically shows an analysis chart obtained by subjecting a blood sample of a high-HbF patient to capillary electrophoresis. As shown in these figures, an HbA peak 100, an HbF peak 120, and an HbA2 peak 160 appear in this order in the analysis chart. As can be seen by comparison between these figures, the HbF peak 120 in the chromatograph of Fig. 5 is higher than the HbF peak 120 in the chromatograph of Fig. 4. However, the sizes of the HbA peak 100 and the HbA2 peak 160 in Fig. 4 are almost the same as the sizes of the HbA peak 100 and the HbA2 peak 160 in Fig. 5. It can thus be assumed that the HbF peak 120 in capillary electrophoresis also includes a modified-HbF peak value.

In view of the above observation, in one aspect of the method of measuring HbF of the disclosure, from a chromatogram obtained by subjecting a blood sample to liquid chromatography, the ratio of the hemoglobin F peak to the entire hemoglobin peak is calculated, and the ratio is multiplied by a predetermined factor, to calculate a corrected value of the hemoglobin F peak with respect to the entire hemoglobin peak. More specifically, the predetermined factor is from 1.15 to less than 1.25. The more preferable predetermined factor is 1.2. The calculation of the corrected value is not limited as long as the ratio of the hemoglobin F peak to the entire hemoglobin peak is multiplied by the predetermined factor as a result. For example, by determining a corrected value by multiplying a peak value of hemoglobin F by a predetermined factor, and calculating the ratio of the corrected value to the peak value of the entire hemoglobin peak, the corrected value of the hemoglobin F peak with respect to the entire hemoglobin peak can be calculated.

### EXAMPLES

### (1) Liquid Chromatography Apparatuses

As liquid chromatography apparatuses, cation-exchange chromatography apparatuses of the following Apparatus 1, Apparatus 2, Apparatus 3, and Apparatus 4 were used.

### (1-1) Apparatus 1

As Apparatus 1, ADAMS HA-8180V (commercially available from ARKRAY, Inc.) was used. For Apparatus 1, a Column Unit 80 (commercially available from ARKRAY, Inc.), which is a column packed with 0.35 ml of a hydrophilic polymer containing a methacrylate copolymer as a packing, was used. As reagents, 80A (commercially available from ARKRAY, Inc.) as Eluent A, 80B (commercially available from ARKRAY, Inc.) as Eluent B, and 80CV (commercially available from ARKRAY, Inc.) as Eluent C were used for eluting hemoglobin from the column, and 80H (commercially available from ARKRAY, Inc.) was used as a hemolysis/washing liquid. The compositions and pHs of 80A, 80B, 80CV, and 80H were as shown in Table 1 below. These reagents were passed though the column at a flow rate of 1.7 ml/min.

### (1-2) Apparatus 2

As Apparatus 2, ADAMS HA-8180T (commercially available from ARKRAY, Inc.) was used. For Apparatus 2, a Column Unit 80T (commercially available from ARKRAY, Inc.), which is a column packed with 0.45 ml of a hydrophilic polymer containing a methacrylate copolymer as a packing, was used. As reagents, 80A as Eluent A, 80B as Eluent B, and 80CT (commercially available from ARKRAY, Inc.) as Eluent C were used for eluting hemoglobin from the column, and 80H was used as a hemolysis/washing liquid. The compositions and pHs of 80A, 80B, 80CT, and 80H were as shown in Table 1 below. These reagents were passed though the column at a flow rate of 1.7 ml/min.

### (1-3) Apparatus 3

As Apparatus 3, ADAMS HA-8190V (commercially available from ARKRAY, Inc.) was used. For Apparatus 3, a Column Unit 90 (commercially available from ARKRAY, Inc.), which is a column packed with 0.25 ml of a hydrophilic polymer containing a methacrylate copolymer as a packing, was used. As reagents, 90A (commercially available from ARKRAY, Inc.) as Eluent A, 90B (commercially available from ARKRAY, Inc.) as Eluent B, and 90CV (commercially available from ARKRAY, Inc.) as Eluent C were used for eluting hemoglobin from the column, and 90H (commercially available from ARKRAY, Inc.) was used as a hemolysis/washing liquid. The compositions and pHs of 90A, 90B, 90CV, and 90H were as shown in Table 1 below. These reagents were passed though the column at a flow rate of 4.0 ml/min.

**[Table 1]**

| Reagent | Name | Composition | pH |
|---|---|---|---|
| Eluent A | 80A | Phosphate buffer | 5.35 ± 0.05 |
| | 90A | Phosphate buffer | 5.35 ± 0.15 |
| Eluent B | 80B | Phosphate buffer | 8.05 ± 0.20 |
| | 90B | Phosphate buffer | 8.05 ± 0.25 |
| Eluent C | 80CV | Phosphate buffer | 6.9 to 7.2 |
| | 80CT | Phosphate buffer | 7.0 to 7.4 |
| | 90CV | Phosphate buffer | 7.05 ± 0.25 |
| Hemolysis / washing liquid | 80H | Phosphate buffer containing a surfactant | 7.5 ± 0.1 |
| | 90H | Phosphate buffer containing a surfactant | 7.5 ± 0.2 |

Regarding the elution strength for hemoglobin, Eluent A had the lowest strength, and Eluent B had the highest strength among Eluents A to C.

### (2) Measurement Method by Liquid Chromatography

Measurement of HbF using Apparatus 1, Apparatus 2, and Apparatus 3 was carried out according to a document accompanying the apparatus provided by ARKRAY, Inc. For each of Apparatus 1 and Apparatus 3, the measurement was carried out by both the first mode and the second mode described below.

### (2-1) First Mode

Eluent A was passed through the column to elute HbF and HbA1c, and then Eluent B was passed through the column to completely elute the remaining hemoglobin.

### (2-2) Second Mode

Eluent A was passed through the column to elute HbF and HbA1c, and then Eluent C was passed through the column to elute HbS, HbC, HbE, and HbD, followed by passing Eluent B through the column to completely elute the remaining hemoglobin.

### (3) Capillary Electrophoresis Apparatus

As a capillary electrophoresis apparatus, Capillarys 2 Flexpiercing (Sebia) was used. The measurement of HbF using the capillary electrophoresis apparatus was carried out according to a document accompanying the apparatus provided by Sebia.

### (4) Measurement Results

Using Apparatus 1 to Apparatus 3, and the capillary electrophoresis apparatus, the HbF peak value was measured for 51 whole blood samples.

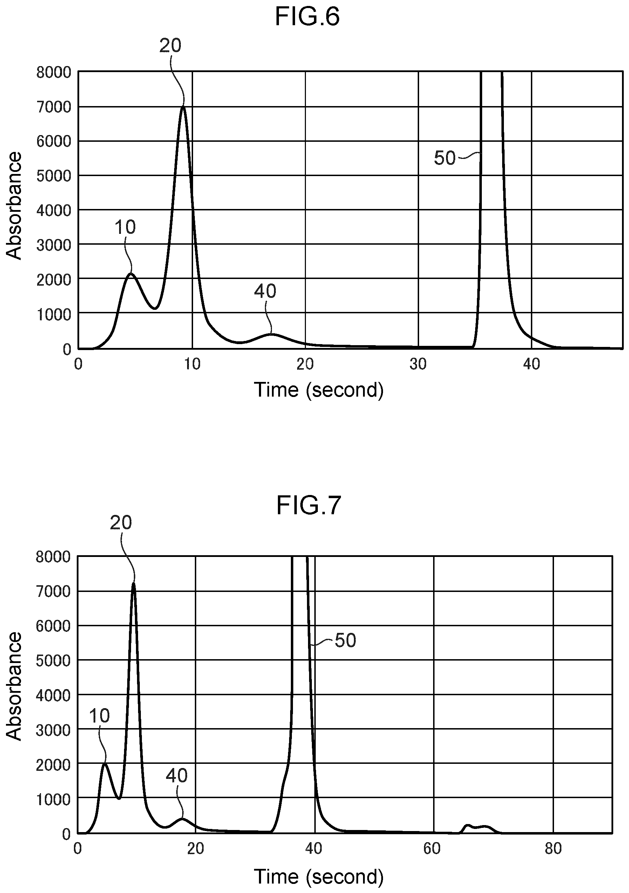

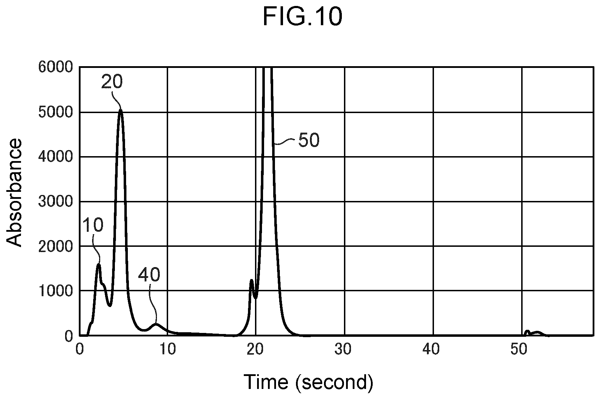

Regarding the liquid chromatography apparatuses, Fig. 6 shows an example of the measurement using Apparatus 1 by the first mode; Fig. 7 shows an example of the measurement using Apparatus 1 by the second mode; Fig. 8 shows an example of the measurement using Apparatus 2; Fig. 9 shows an example of the measurement using Apparatus 3 by the first mode; and Fig. 10 shows an example of the measurement using Apparatus 3 by the second mode. It was shown that the HbF peak 20, and the composite peak 10 of the HbA1a peak and the HbA1b peak, can be separated from each other by any of the apparatuses.

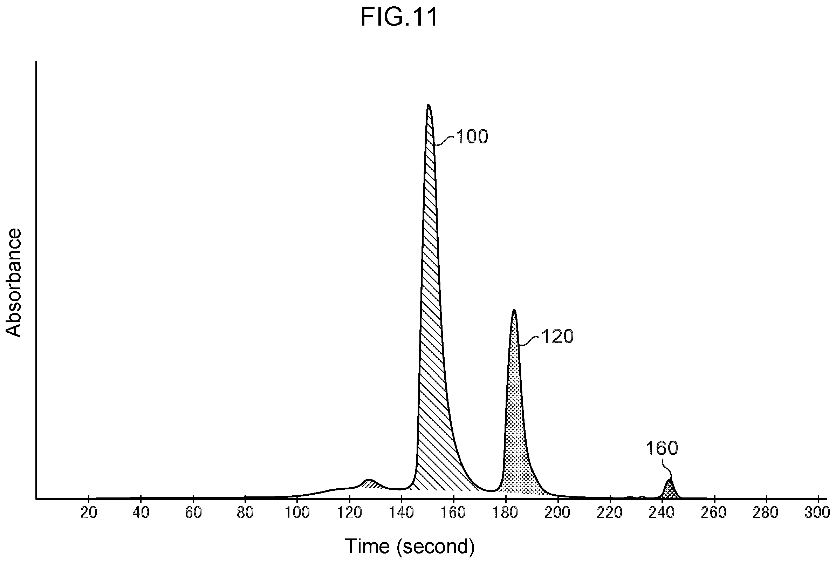

Fig. 11 shows an example of the measurement using the capillary electrophoresis apparatus. The ratio of the HbF peak 120 to the total of the hemoglobin peaks in the analysis chart of Fig. 11 was found to be clearly higher than the ratio of the HbF peak 20 to the total of the hemoglobin peaks analyzed using each liquid chromatography apparatus.

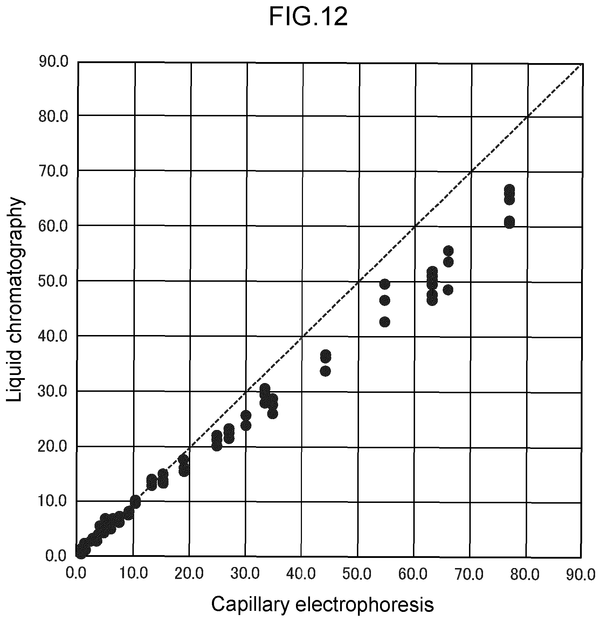

Fig. 12 is a graph illustrating a correlation between the HbF peak value obtained by the measurement using each liquid chromatography apparatus and the HbF peak value obtained by the measurement using the capillary electrophoresis apparatus, wherein the measurement was carried out for the same sample. The abscissa of the graph represents the HbF peak value obtained by the capillary electrophoresis apparatus, and the ordinate of the graph represents the HbF peak value obtained by the liquid chromatography apparatus. The dotted line in the graph is the line of
y = x
wherein x is the abscissa, and y is the ordinate. From this graph, it was found that the HbF peak value obtained by the capillary electrophoresis apparatus was higher than the HbF peak value obtained by each liquid chromatography apparatus.

Table 2 below shows the ratio of the HbF peak value obtained by the capillary electrophoresis apparatus to the HbF peak value obtained by each liquid chromatography apparatus. It was found that the HbF peak value obtained by the capillary electrophoresis apparatus is 1.18 to 1.23 times the HbF peak value obtained by each liquid chromatography apparatus.

**[Table 2]**

| Apparatus | Mode | Ratio |
|---|---|---|
| Apparatus 1 | First mode | 1.20 |
| | Second mode | 1.19 |
| Apparatus 2 | - | 1.18 |
| Apparatus 3 | First mode | 1.18 |
| | Second mode | 1.23 |

Thus, it was shown that, by multiplying an HbF peak value obtained by a liquid chromatography apparatus by a factor from 1.15 to less than 1.25, preferably a factor from 1.18 to 1.23, more preferably a factor of 1.2, a more accurate HbF peak value can be measured similarly to a case where the value is obtained by a capillary electrophoresis apparatus.

### Industrial Applicability

The invention is applicable to measurement of HbF in a blood sample using liquid chromatography.

## Claims

1. A method of measuring hemoglobin F, the method comprising:
calculating, from a chromatogram obtained by subjecting a blood sample to liquid chromatography, a ratio of a peak value of a hemoglobin F peak (20) to a peak value of an entire hemoglobin peak; and
calculating a corrected value of the hemoglobin F peak (20) with respect to the entire hemoglobin peak by multiplying the ratio by a predetermined factor, **characterized in that** the predetermined factor is calculated by
measuring, for a series of samples, a hemoglobin F peak value obtained by liquid chromatography and a hemoglobin F peak value obtained by capillary electrophoresis;
calculating the predetermined factor corresponding to the hemoglobin F peak value obtained by capillary electrophoresis divided by the hemoglobin F peak value obtained by liquid chromatography.

2. The method of measuring hemoglobin F according to claim 1, wherein the predetermined factor is from 1.15 to less than 1.25.

3. The method of measuring hemoglobin F according to claim 2, wherein the predetermined factor is 1.2.

4. The method of measuring hemoglobin F according to any one of claim 1 to claim 3, wherein the liquid chromatography is cation-exchange chromatography.

## Patentansprüche

1. Verfahren zum Messen von Hämoglobin F, wobei das Verfahren umfasst:
Berechnen eines Verhältnisses eines Peak-Werts eines Hämoglobin-F-Peaks (20) zu einem Peak-Wert eines gesamten Hämoglobin-Peaks aus einem Chromatogramm, das erhalten wird, indem eine Blutprobe einer Flüssigchromatographie unterzogen wird; und
Berechnen eines korrigierten Wertes des Hämoglobin-F-Peaks (20) in Bezug auf den gesamten Hämoglobin-Peak durch Multiplizieren des Verhältnisses mit einem vorbestimmten Faktor,
**dadurch gekennzeichnet, dass** der vorbestimmte Faktor
durch Messen eines Hämoglobin-F-Peak-Werts, der durch Kapillarelektrophorese erhalten wird, berechnet wird;
Berechnen des vorbestimmten Faktors entsprechend dem durch Kapillarelektrophorese erhaltenen Hämoglobin-F-Peak-Wert geteilt durch den durch Flüssigchromatographie erhaltenen Hämoglobin-F-Peak-Wert.

2. Verfahren zum Messen von Hämoglobin F nach Anspruch 1, wobei der vorbestimmte Faktor zwischen 1,15 und weniger als 1,25 liegt.

3. Verfahren zum Messen von Hämoglobin F nach Anspruch 2, wobei der vorbestimmte Faktor 1,2 beträgt.

4. Verfahren zum Messen von Hämoglobin F nach einem von Anspruch 1 bis Anspruch 3, wobei die Flüssigchromatographie eine Kationenaustauschchromatographie ist.

## Revendications

1. Procédé de mesure de l'hémoglobine F, le procédé comprenant :
le calcul, à partir d'un chromatogramme obtenu en soumettant un échantillon de sang à une chromatographie liquide, d'un rapport entre une valeur de pic d'un pic d'hémoglobine F (20) et une valeur de pic d'un ensemble de pics d'hémoglobine ; et
le calcul d'une valeur corrigée du pic d'hémoglobine F (20) par rapport à l'ensemble de pics d'hémoglobine en multipliant le rapport par un facteur prédéterminé, **caractérisé en ce que** le facteur prédéterminé est calculé par la valeur de pic d'hémoglobine F obtenue par électrophorèse capillaire ;
le calcul du facteur prédéterminé correspondant à la valeur de pic d'hémoglobine F obtenue par électrophorèse capillaire divisée par la valeur de pic d'hémoglobine F obtenue par chromatographie liquide.

2. Procédé de mesure de l'hémoglobine F selon la revendication 1, dans lequel le facteur prédéterminé est de 1,15 à moins de 1,25.

3. Procédé de mesure de l'hémoglobine F selon la revendication 2, dans lequel le facteur prédéterminé est de 1,2.

4. Procédé de mesure de l'hémoglobine F selon l'une quelconque de la revendication 1 à la revendication 3, dans lequel la chromatographie liquide est une chromatographie échangeuse de cations.
